(19) Europäisches Patentamt
European Patent Office
Office européen des brevets

(11) Veröffentlichungsnummer : **0 411 408 B1**

# (12) EUROPÄISCHE PATENTSCHRIFT

(45) Veröffentlichungstag der Patentschrift : **03.11.93 Patentblatt 93/44**

(51) Int. Cl.$^5$ : **C07D 231/12,** A01N 43/56

(21) Anmeldenummer : **90113914.7**

(22) Anmeldetag : **20.07.90**

## (54) Monoklines Metazachlor und Verfahren zu seiner Herstellung.

(30) Priorität : **29.07.89 DE 3925253**

(43) Veröffentlichungstag der Anmeldung : **06.02.91 Patentblatt 91/06**

(45) Bekanntmachung des Hinweises auf die Patenterteilung : **03.11.93 Patentblatt 93/44**

(84) Benannte Vertragsstaaten : **AT BE CH DE DK ES FR GB IT LI NL**

(56) Entgegenhaltungen :
**EP-A- 0 012 216**
**EP-A- 0 023 287**
**DE-A- 2 648 008**
**DE-A- 2 830 764**

(73) Patentinhaber : **BASF Aktiengesellschaft**
**Carl-Bosch-Strasse 38**
**D-67063 Ludwigshafen (DE)**

(72) Erfinder : **Keil, Michael, Dr.**
**Fontanestrasse 4**
**D-6713 Freinsheim (DE)**
Erfinder : **Girgensohn, Björn, Dr.**
**Neckarpromenade 38**
**D-6800 Mannheim 1 (DE)**
Erfinder : **Synnatschke, Gotthard, Dr.**
**Thomas-Mann-Strasse 24**
**D-6700 Ludwigshafen (DE)**
Erfinder : **Wigger, August, Dr.**
**Rehbachstrasse 22**
**D-6708 Neuhofen (DE)**
Erfinder : **Ziegler, Hans, Dr.**
**Pfalzring 91**
**D-6704 Mutterstadt (DE)**
Erfinder : **Gückel, Walter, Dr.**
**Parkstrasse 21**
**D-6703 Limburgerhof (DE)**

**EP 0 411 408 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft monoklines, bei 76°C schmelzendes 2-Chlor-(2′,6'dimethyl-N-pyrazol-1-yl-methyl)-acetanilid der Formel I

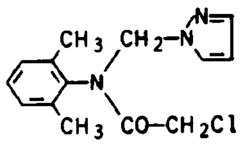

I

sowie Verfahren zur Herstellung dieser Modifikation, deren Verwendung als Herbizid und herbizide Mittel, die diesen Wirkstoff, vorzugsweise 0,1 bis 95 Gew.-% und übliche inerte Zusatzstoffe enthalten.

Der wichtige herbizide Wirkstoff 2-Chlor-(2',6'-dimethyl-N-pyrazol-1-yl-methyl)-acetanilid I (Kurzbezeichnung Metazachlor) schmilzt soweit bisher bekannt ist, in einem Bereich von 78-83°C und kristallisiert in einer triklinen Kristallform. Man erhält diese Kristallform IA nach den in DE-A 2 648 008, DE-A 2 830 764 und EP-A 12216 beschriebenen Methoden durch Kristallisation von I aus einem unpolaren oder wenig polaren Lösungsmittel wie Cyclohexan oder Toluol.

Diese bekannte Modifikation IA von Metazachlor, welches in Form konzentrierter wäßriger Suspensionen in den Handel gebracht wird, hat nun allerdings den Nachteil, daß sie häufig Agglomerate bildet.

Die Mittel können dann nicht mehr gleichmäßig oder sogar überhaupt nicht mehr versprüht werden.

Im Bestreben, diesem Mangel abzuhelfen, wurde eine monokline Modifikation von I gefunden, die bei 76°C schmilzt.

Man erhält diese monokline Modifikation IB, wenn man eine wäßrige schwefelsaure Metazachlor-Lösung in Gegenwart eines mit Wasser mischbaren polaren inerten organischen Lösungsmittels bei Temperaturen von 0°C bis 50°C mit Wasser versetzt und den dabei gebildeten Festkörper nach vollständiger Kristallisation in üblicher Weise isoliert.

Bei diesem Verfahren ist es für die Kristallisation unerheblich in welcher Reihenfolge die Wirkstofflösung, das organische Lösungsmittel und Wasser miteinander gemischt werden.

Animpfen mit Kristallen der Modifikation IB ist nicht zwingend, erweist sich aber im allgemeinen als vorteilhaft.

Als protisch polare inerte Lösungsmittel eignen sich insbesondere Alkohole wie Methanol, Ethanol, Isopropanol, n-Propanol, 1-Butanol, 2-Butanol, Isobutanol, tert.-Butanol, Ethylenglykol, Propylenglykol, Propan-1,3-diol und Butandiole, Ketone wie Aceton und Butan-2-on, Ether wie Tetrahydrofuran und 1,4-Dioxan, Amide wie Dimethylformamid und N-Methylpyrrolidon, sowie Dimethylsulfoxid und Diethylenglykol.

Die zur Herstellung von Metazachlor in der Modifikation IB in Mengen zwischen 0,01 bis 50 Gew.-% erforderlichen Impfkristalle können erstmalig z.B. durch Umkristallisieren von Metazachlor der Modifikation IA in den zuvor genannten polaren organischen Lösungsmitteln erhalten werden.

Eine weitere Möglichkeit zur Herstellung von Metazachlor der Modifikation IB besteht darin, daß man wäßrige Suspensionen von Metazachlor der Modifikation IA bei pH-Werten zwischen 0 und 10 gegebenenfalls unter Zusatz eines oder mehrerer der oben genannten wasserlöslichen organischen Lösungsmittel bei Temperaturen von 0 bis 45°C mit 0,01 bis 50 Gew.-% an Impfkristallen der Modifikation IB versetzt und zwischen 0,1 und 20 Stunden rührt. Hierbei findet eine Umwandlung von Modifikation IA in Modifikation IB statt.

In analog zu betrachtender Weise gelingt diese Umwandlung auch bei der Vermahlung einer wäßrigen Suspension von Modifikation IA in Gegenwart der genannten organischen Lösungsmittel unter Zusatz von Impfkristallen der Modifikation IB.

Charakteristische physikalische Daten zur Unterscheidung der beiden Modifikationen sind in der folgenden Tabelle zusammengestellt.

Tabelle

| Meßmethode | Dimension | IA | IB |
| --- | --- | --- | --- |
| Differenzial-thermoanalyse | °C | 79 | 76 |
| IR-Spektroskopie* | $cm^{-1}$ | 3160, 1300 | 1360, 780 |
| Röntgeninterferenz-diagramm (Brechungswinkel 2 θ) | Grad | 8,2; 8,4 | 9,9; 12,3 |
| 13C-Festkörper-NMR-Spektroskopie* (gegen Adamantan) | δ in ppm | 62,5; 137,1 | 51,4; 139,1 |
| Modifikation aus Röntgenstruktur-analyse | - | triklin | monoklin |
| Mikroskopie (Kristallform) | - | "amorph" | "sargdeckel"förmig |

*ausgewählte Signale

Die Erfindung wirkt sich folgendermaßen aus:

Formulierte Ware mit Metazachlor der bislang bekannten Modifikation IA verändert sich während der Lagerung. Die Korngröße der suspendierten Teilchen nimmt kontinuierlich zu, in der formulierten Ware äußert sich dies in Brockenbildung bis hin zu völligem Festwerden des zuvor flüssigen Produkts, so daß ein gleichmäßiges Austragen der Ware nicht mehr gewährleistet ist.

Formulierte Ware mit Metazachlor der Modifikation IB zeigt dieses ungewünschte Verhalten nicht und läßt sich auch nach längerer Lagerung einwandfrei ausbringen.

Im Rahmen dieser Untersuchungen wurde eine weitere metastabile bei 84°C schmelzende Metazachlor-Modifikation IC gefunden. Man erhält diese Modifikation durch Erwärmen von Metazachlor der Modifikation IA auf 84°C und Anreiben der gebildeten Schmelze.

Beim Stehen wandelt sich die Metazachlor-Modifikation IC mit einer Halbwertszeit von ca. 5-Tagen in die bei 76°C schmelzende Modifikation IB um.

Die folgenden Beispiele verdeutlichen das erfindungsgemäße Verfahren:

1. Fällung einer sauren wäßrigen Metazachlorlösung unter Animpfen mit Modifikation IB.

   a) 1 250 ml Wasser und 22 ml Methanol werden bei 25°C vorgelegt. Zum Animpfen werden 3,8 g Metazachlor Modifikation IB zugesetzt. Nun gibt man langsam unter Rühren 62,5 g einer 40 %-igen Metazachlorlösung in 60 %-iger Schwefelsäure zu, rührt eine Stunde und gibt anschließend schnell weitere 562,5 g der Metazachlorlösung zu. Nach vollständiger Fällung wird der Niederschlag isoliert, mit Wasser gewaschen und getrocknet. Man erhält 247 g Metazachlor der Modifikation IB.

   b) Eine Lösung von 63 g Metazachlor der Modifikation IA in 90 g 60 %iger Schwefelsäure wird unter Rühren bei 40°C mit 7,6 g Methanol und 51 g Wasser versetzt. Nach zugabe von 3 g Metazachlor Modifikation IB gibt man innerhalb von zwei Stunden 133 ml und anschließend schnell weitere 113 ml Wasser zu. Es wird zwei Stunden nachgerührt, nach Absaugen mit 200 ml Wasser gewaschen und getrocknet. Man erhält 62,3 g Metazachlor der Modifikation IB.

2. Herstellung von Metazachlor Modifikation IB durch Umwandlung der Kristallmodifikation IA in wäßriger

Suspension.

Eine Suspension von 10 g Metazachlor Modifikation IA in 127 g 15 %-iger Schwefelsäure und 2 g Methanol wird mit 1 g Modifikation IB versetzt, 20 Stunden bei 20°C gerührt und nach dem Absaugen mit 500 ml Wasser gewaschen. Man erhält Metazachlor der Modifikation IB.

3. Herstellung von Metazachlor Modifikation IB durch Kristallisation aus methanolisch-wäßriger Lösung

Eine Mischung aus 2,2 l Methanol und 1 500 g Metazachlor Modifikation IA wird bei 50°C in Lösung gebracht. Man läßt langsam auf Raumtemperatur kommen und gibt zu der teilweise auskristallisierenden Mischung langsam 5,2 l Wasser. Nach Absaugen und Waschen mit 1 l Wasser erhält man 1 485 g Metazachlor der Modifikation IB.

## Patentansprüche

1. Monoklines, bei 76°C schmelzendes 2-Chlor-(2',6'-dimethyl-N-pyrazol-1-yl-methyl)-acetanilid der Formel I

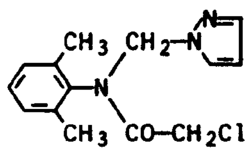

I

2. Verfahren zur Herstellung kristalliner Massen der Verbindung I nach Anspruch 1, dadurch gekennzeichnet, daß man die Verbindung I aus einem polaren inerten organischen Lösungsmittel umkristallisiert und nach vollständiger Kristallisation den Festkörper in üblicher Weise isoliert.

3. Verfahren zur Herstellung kristalliner Massen der Verbindung I nach Anspruch 1, dadurch gekennzeichnet, daß man 2-Chlor-(2',6'-dimethyl-N-pyrazol-1-yl-methyl)-acetanilid aus wäßriger schwefelsaurer Lösung bei Temperaturen von 0 bis 50°C in Gegenwart eines polaren mit Wasser mischbaren inerten organischen Lösungsmittels kristallisiert.

4. Verfahren zur Herstellung kristalliner Massen der Verbindung I nach Anspruch 1, dadurch gekennzeichnet, daß man eine wäßrige Suspension der Verbindung I in der bei 79°C schmelzenden triklinen Kristallmodifikation von I mit einem polaren mit Wasser mischbaren inerten organischen Lösungsmittel in Gegenwart von Kristallen der Verbindung I in der bei 76°C schmelzenden monoklinen Kristallmodifikation bei 0°C bis 45°C vermahlt.

5. Herbizides Mittel, enthaltend übliche inerte Zusatzstoffe und die Kristallmodifikation von I gemäß Anspruch 1.

6. Herbizides Mittel nach Anspruch 5, dadurch gekennzeichnet, daß es 0,1 bis 95 Gew.-% der bei 76°C schmelzenden monoklinen Kristallmodifikation von I enthält.

7. Verfahren zur Bekämpfung unerwünschten Pflanzenwachstum, dadurch gekennzeichnet, daß man die Samen, die Pflanzen und/oder deren Lebensraum mit einer herbizid wirksamen Menge der Kristallmodifikation von I gemäß Anspruch 1 behandelt.

## Claims

1. Monoclinic 2-chloro-(2',6'-dimethyl-N-pyrazol-1-ylmethyl)-acetanilide of the formula I

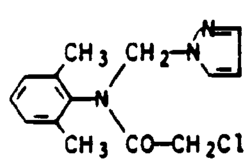

I

which melts at 76°C.

2. A process for the preparation of a crystalline compound I as claimed in claim 1, wherein the compound I is recrystallized from a polar inert organic solvent and the solid is isolated in a conventional manner after crystallization is complete.

3. A process for the preparation of a crystalline compound I as claimed in claim 1, wherein 2-chloro-(2',6'-dimethyl-N-pyrazol-1-ylmethyl)-acetanilide is crystallized from aqueous solution containing sulfuric acid, at from 0 to 50°C, in the presence of a polar water-miscible inert organic solvent.

4. A process for the preparation of a crystalline compound I as claimed in claim 1, wherein an aqueous suspension of the compound I in the triclinic crystalline modification of I, which modification melts at 79°C, is milled, at from 0 to 45°C, with a polar water-miscible inert organic solvent in the presence of crystals of compound I in the monoclinic crystalline modification melting at 76°C.

5. A herbicide containing conventional inert additives and the crystalline modification of I as claimed in claim 1.

6. A herbicide as claimed in claim 5, which contains from 0.1 to 95% by weight of the monoclinic crystalline modification of I, which modification melts at 76°C.

7. A method for controlling undesirable plant growth, wherein the seeds, the plants and/or their habitat are or is treated with a herbicidally effective amount of the crystalline modification of I as claimed in claim 1.

## Revendications

1. 2-Chloro-(2', 6'-diméthyl-N-pyrazol-1-yl-méthyl)-acétanilide monoclinique fondant à 76 degrés C, de formule I

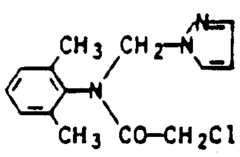

I

2. Procédé de préparation de masses cristallisées du composé I, selon la revendication 1, caractérisé par le fait que l'on recristallise le composé I à partir d'un solvant organique inerte, polaire, et après cristallisation complète, on isole les solides de manière usuelle.

3. Procédé de préparation de masses cristallisées du composé I, selon la revendication 1, caractérisé par le fait que l'on cristallise du 2-chloro-(2', 6'-diméthyl-N-pyrazol-1-yl-méthyl)-acétanilide à partir d'une solution sulfurique aqueuse, à des températures de 0 à 50 degrés C, en présence d'un solvant organique inerte, polaire, miscible avec l'eau.

4. Procédé de préparation de masses cristallisées du composé I, selon la revendication 1, caractérisé par le fait que l'on broie, entre 0 degré C et 45 degrés C, une suspension aqueuse du composé I sous forme de la modification cristalline triclinique, fondant à 79 degrés C, avec un solvant organique inerte, polaire, miscible avec l'eau, en présence de cristaux du composé I sous forme de la modification cristalline monoclinique, fondant à 76 degrés C.

5. Agent herbicide contenant des additifs inertes usuels et la modification cristalline de I, selon la revendication 1.

6. Agent herbicide selon la revendication 5, caractérisé par le fait qu'il contient 0,1 à 95 % en poids de la modification cristalline monoclinique de I, fondant à 76 degrés C.

7. Procédé de lutte contre la croissance des plantes indésirables, caractérisé par le fait que l'on traite les semences, les plantes et/ou leur biotope avec une quantité efficace du point de vue herbicide de la modification cristalline de I selon la revendication I.